# EUROPEAN PATENT SPECIFICATION

(11) **EP 4 135 668 B1**
(45) Date of publication and mention of the grant of the patent: **17.04.2024**
(21) Application number: 21725812.8
(22) Date of filing: 14.04.2021
(51) Int. Cl.: A61K 9/16, A61K 31/454, A61P 35/00

(54) **SOLID ORAL DOSAGE FORM COMPRISING POMALIDOMIDE**
FESTE ORALE DARREICHUNGSFORM MIT POMALIDOMID
FORME POSOLOGIQUE ORALE SOLIDE COMPRENANT DU POMALIDOMIDE

(30) Priority: 15.04.2020 EP 20169699; 15.04.2020 PT 2020116261
(43) Date of publication of application: 22.02.2023
(73) Proprietor: Tecnimede, Sociedade Técnico-Medicinal, SA, 2710-089 Sintra (PT)
(72) Inventor: FERNANDES MARQUES RAMOS FRANCO FRAZÃO, Joana Maria, 1600-545 Lisboa (PT); SILVA SERRA, João Pedro, 2710-089 Sintra (PT)
(74) Representative: Patentree
(86) International application number: PCT/IB2021/053073
(87) International publication number: WO 2021/209919

(56) References cited:
- WO-A1-2018/024646
- WO-A1-2018/150435
- CN-A- 104 042 590

## Description

### TECHNICAL FIELD

The present disclosure relates to the field of pharmaceutical technology, in particular to the formulation of new pharmaceutical dosage forms of pomalidomide for therapeutic use.

### BACKGROUND

Pomalidomide is the international non-proprietary name (INN) of 4- amino-2-(2,6-dioxopiperidine-3-yl)isoindoline-l,3-dione with the following structure:

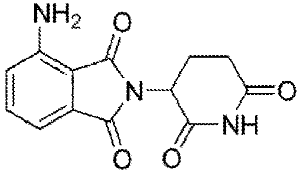

Pomalidomide is currently registered and marketed in many countries under the brand names IMNOVID^{®} and POMALYST^{®}. Pomalidomide is used in monotherapy or in combination with dexamethasone for the treatment of multiple myeloma.

Document WO 9803502 discloses a method of synthesizing pomalidomide. Document CN 104042590 A discloses capsule compositions comprising pomalidomide, anhydrous lactose, dextrin, croscarmellose sodium and polyethylene glycol PEG 4000.

The prosecution history of EP 2 391 355 (Celgene) discloses that pharmaceutical compositions of pomalidomide comprising several excipients such as anhydrous dibasic calcium phosphate, starch (corn starch and pregelatinized), lactose anhydrous, microcrystalline cellulose, croscarmellose sodium, starch glycolate and/or sodium stearyl fumarate or magnesium stearate presented compatibility problems, i.e. were unstable after two weeks. Compositions of pomalidomide comprising anhydrous lactose, microcrystalline cellulose, croscarmellose sodium and magnesium stearate were initially deemed to be suitable for the production of pharmaceutical products but later evidenced unsatisfactory storage stability.

Furthermore, the European Public Assessment report (EPAR) for IMNOVID^{®} discloses that the initial attempt to formulate IMNOVID oral composition contained pomalidomide, anhydrous lactose, microcrystalline cellulose, croscarmellose sodium and magnesium stearate. However, this initial composition was deemed unsuitable due to processing issues and during the development of the formulation, a number of qualitative alterations were implemented with the purpose of overcoming the limitations. The removal of anhydrous lactose and its direct substitution by dibasic calcium phosphate is singled out.

Pomalidomide is a Class IV compound according to the Biopharmaceutics Classification System (BCS Class IV) meaning that it is characterized as a low aqueous solubility and low permeability active compound. Class IV compounds' poor bioavailability makes it challenging for pharmaceutical formulators to develop compositions comprising these compounds. Absorption is characteristically poor and an increased plasmatic variability is expected across the pH range (1.2 to 6.8).

Systemic absorption of most drug products consists of a succession of rate processes. These processes include: (i) disintegration of the drug product and subsequent release of the drug; (ii) dissolution of the drug in an aqueous environment; and (iii) absorption across cell membrane into the systemic circulation. During the process of drug disintegration, dissolution and absorption, the rate at which the drug reaches the circulatory system is determined by the slowest step in the sequence. The slowest step in a series of kinetic process is called the rate-limiting step, except for sustained release or prolonged-action products, disintegration of a solid drug product is usually more rapid than dissolution and drug absorption. For drugs that have very poor aqueous solubility, for example pomalidomide, the rate at which the drug dissolves (dissolution) is often the slowest step and therefore exerts a rate-limiting effect on drug bioavailability.

In biologic systems, drug dissolution in an aqueous medium is an important condition for systemic absorption and subsequent bioavailability. The rate at which drug with poor aqueous solubility dissolve from an intact or disintegrated solid dosage form into the gastro intestinal tract often controls the rate of systemic absorption of the drug.

Pomalidomide is an immunomodulatory drug which is used for the treatment of multiple myleoma.

Taking into consideration the abovementioned, it is expected that pomalidomide's solubility may limit its dissolution rate and dissolution extension. For a drug substance that has a poor aqueous solubility, the rate at which it dissolves in the gastrointestinal fluids is often the slowest step, and therefore the bioavailability of that drug is said to be dissolution-rate limited.

According to the results obtained during the pharmaceutical development, for immediate release drug products that present low aqueous solubility which impacts the dissolution rate, time points of 60-minutes are considered the most suitable indicators of complete drug release.

The main goal of performing a 60-minutes time point dissolution test is to ensure that a complete release of the drug is achieved. Complete drug release is deemed to have occurred if, after 60 minutes, at least 90% of the pomalidomide is dissolved. Achieving this result means the drug dissolution profile is consistent with *in vivo* dissolution performance and hence implies in vivo drug efficacy and safety. This tight specification for a highly discriminating *in vitro* test is to ensure that the desired therapeutic or pharmacological effect is achieved. The therapeutic or pharmacological effect is measured *in vivo* through the pharmacokinetic parameters AUC and Cₘₐₓ.

Plasma concentration time curve is generally used to assess the rate and extent of drug absorption. AUC, the area under the concentration time curve, reflects the extent of drug exposure and is related to the extent of drug dissolution. Cₘₐₓ, the maximum plasma concentration of peak exposure, and the time to maximum plasma concentration, tₘₐₓ, are parameters that are influenced by absorption rate and are related to the dissolution rate.

### GENERAL DESCRIPTION

The present disclosure relates to new pharmaceutical dosage forms of pomalidomide for therapeutic use.

In an embodiment, the present disclosure relates to an immediate release pharmaceutical composition comprising
0.10 % to 3.00 % (wt/wt) of pomalidomide;
40.00 % to 93.00 % (wt/wt) of anhydrous lactose as a diluent;
6.00 % to 25.00 % (wt/wt) of hypromellose as a binder.

In an embodiment, the pharmaceutical composition further comprises 5% to 20% (wt/wt) of at least a further diluent.

In an embodiment, the pharmaceutical composition further comprises 2% to 10% (wt/wt) of disintegrant.

In an embodiment, the pharmaceutical composition further comprises 0.2% to 2% (wt/wt) of lubricant.

In an embodiment, the further diluent ranges from 10% to 20% (wt/wt), preferably from 10% to 15% (wt/wt).

In an embodiment, the pharmaceutical composition comprises 1 mg to 4 mg of pomalidomide.

In an embodiment, the amount of pomalidomide is from 0.2% to 2% (wt/wt), preferably from 0.3% to 1.5% (wt/wt).

In an embodiment, the amount of anhydrous lactose ranges from 50% to 90% (wt/wt).

In an embodiment, the amount of hypromellose ranges from 8 % to 22 % (wt/wt).

In an embodiment, the lubricant is selected from: magnesium stearate, sodium stearyl fumarate, calcium stearate, stearic acid, stearic acid, glyceryl behenate, hexanedioic acid, hydrogenated vegetable oil, glycerine fumarate, or mixtures thereof.

In an embodiment, the disintegrant(s) is selected from: sodium croscarmellose, sodium carboxymethyl cellulose, sodium starch glycolate, crospovidone, or mixtures thereof.

In an embodiment, the further diluent is microcrystalline cellulose.

In an embodiment, the pharmaceutical composition comprises: 1.43% (wt/wt) pomalidomide, 75.57% (wt/wt) anhydrous lactose, 12.5 % (wt/wt) microcrystalline cellulose, 10% (wt/wt) hypromellose, and 0.5% (wt/wt) magnesium stearate.

In an embodiment, the pharmaceutical composition comprises: 0.36% (wt/wt) pomalidomide, 76.64% (wt/wt) anhydrous lactose, 12.5% (wt/wt) microcrystalline cellulose, 10% (wt/wt) Hypromellose, and 0.5% (wt/wt) magnesium stearate.

In an embodiment, the pharmaceutical composition comprises: 1.43% (wt/wt) pomalidomide, 83.07% (wt/wt) anhydrous lactose, 5 % (wt/wt) sodium croscarmellose, 10% (wt/wt) Hypromellose, and 0.5% (wt/wt) magnesium stearate.

In an embodiment, the pomalidomide has a particle size characterized by a D50 ranging from 3µm to 20µm, preferably ranging from 5µm to 10µm.

In an embodiment, the pharmaceutical is a capsule, preferably a hard shell capsule.

In an embodiment, the pharmaceutical ingredient percentage is not more than 5 wt.% of total capsule content.

In an embodiment, the pharmaceutical composition is for use in the treatment of cancer, preferably multiple myeloma.

In an embodiment, the present disclosure relates to a solid pharmaceutical dosage form comprising the composition of the present disclosure in the form of a capsule, preferably a hard-shell capsule.

In an embodiment, the pharmaceutical composition of the present disclosure comprises hydroxypropyl methylcellulose (hypromellose).

In an embodiment, the pharmaceutical compounds of the present disclosure has stability profile, dissolution profile, immediate release profile despite containing anhydrous lactose and Hypromellose, in particular, its dissolution profile at 60-minutes time point.

### BRIEF DESCRIPTION OF THE DRAWINGS

The following figures provide preferred embodiments for illustrating the disclosure and should not be seen as limiting the scope of invention.

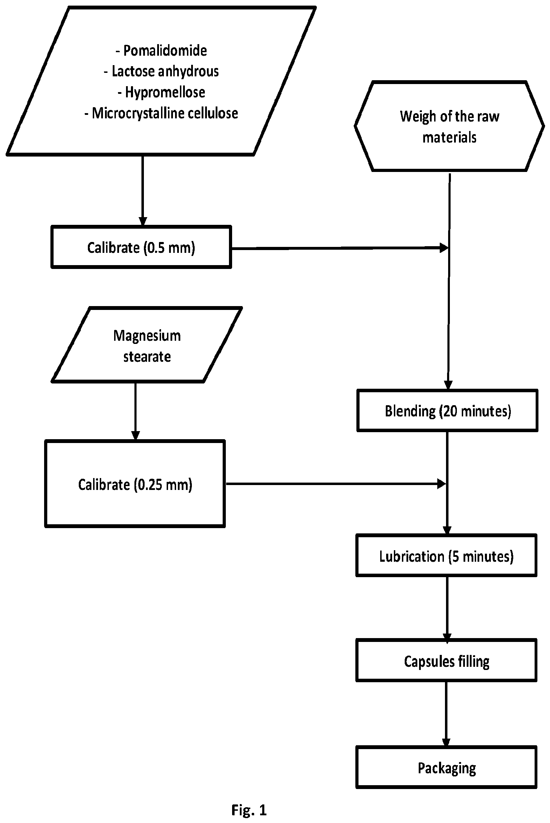

**Figure 1** shows a manufacturing process flowchart of pharmaceutical compositions according to table 4.

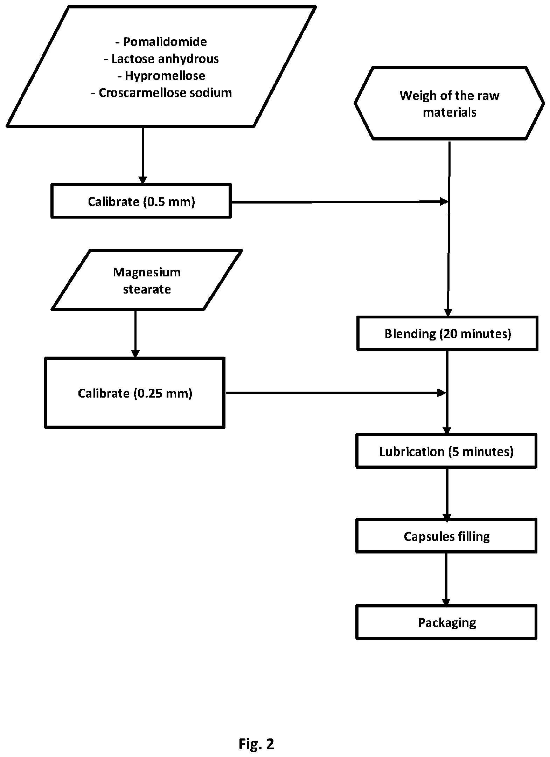

**Figure 2** shows a manufacturing process flowchart of pharmaceutical compositions 2c and 2d.

### DETAILED DESCRIPTION OF THE INVENTION

The present disclosure relates to new pharmaceutical dosage forms of pomalidomide for therapeutic use.

In an embodiment, the pharmaceutical composition of the present disclosure comprises hydroxypropyl methylcellulose (hypromellose).

In an embodiment, the amount of hypromellose ranges from 6% to 25% (wt /wt), preferably from 7% to 23% (wt /wt), more preferably from 8% to 22% (wt /wt).

Hypromellose is well known in the art and its use as a binder usually corresponds to a total % by weight of a composition ranging from 2% to 5%. When present in larger amounts, 10% to 80% by weight of a composition, it is known to form a matrix resulting in the delayed release of active substances. In the present disclosure, hypromellose is used in an amount that is expected to contribute to a slower drug release, however, it acts as a fundamental component of immediate release compositions of poorly soluble active ingredients. Despite comprising an active ingredient with documented low solubility, the compositions described in the present disclosure possess an advantageous immediate release profile with high dose dissolution at the 60 minutes time point.

In an embodiment, the pharmaceutical compositions of the present disclosure also includes anhydrous lactose as diluent.

In an embodiment, the amount of diluents range from 40% to 93% (wt/wt), preferably from 50% to 92%, more preferably from 60% to 90% (wt/wt). The compositions obtained are stable and with no detectable adverse effect (with regard to either its stability or industrial processing) attributable to the presence of anhydrous lactose .

In an embodiment, the pharmaceutical composition further comprises additional pharmaceutical excipients namely, at least a further diluent(s), lubricant(s) and disintegrant(s). In the context of the present disclosure, the inclusion of each of these excipients shall be understood as including a single compound as well as mixtures of two or more compounds of that class.

In an embodiment, the pharmaceutically acceptable further diluent(s) according to the present disclosure include microcrystalline cellulose, kaolin, magnesium oxide, calcium sulfate, citric acid, tartaric acid, fumaric acid, co-polymers of vinyl pyrrolidone and vinyl acetate, co-polymers of polyethylene glycol, and mixtures thereof.

In an embodiment, the further diluent(s) make up from 5% to 20% (wt/wt), preferably from 10% to 20% (wt/wt) and, more preferably from 10% to 15% (wt/wt).

In an embodiment, the preferred further diluent is microcrystalline cellulose.

In an embodiment, pharmaceutically acceptable lubricant(s) according to the present disclosure includes magnesium stearate, sodium stearyl fumarate, calcium stearate, stearic acid,
glyceryl behenate, hexane dioic acid, hydrogenated vegetable oil, glycerin fumarate and mixtures thereof.

In an embodiment, the amount of lubricant(s) ranges from 0.2% to 2% (wt/wt), preferably from 0.3% to 1% (wt /wt).

In an embodiment, the preferred lubricant is sodium magnesium stearate.

In an embodiment, pharmaceutically acceptable disintegrant(s) according to the present disclosure includes croscarmellose, sodium carboxymethyl cellulose, sodium starch glycolate, crospovidone and mixtures thereof.

In an embodiment, the amount of disintegrant(s) ranges from 2% to 10% (wt/wt), preferably from 3% to 8% (wt /wt).

In an embodiment, the preferred disintegrant is croscarmellose sodium.

In an embodiment, the pomalidomide is micronized prior to the formulation.

In an embodiment, the pharmaceutical composition comprises pomalidomide with a particle size characterized by a D₅₀ ranging from 3µm to 20µm, preferably ranging from 5µm to 10µm.

In an embodiment, the oral dosage form of the pharmaceutical composition is a capsule, preferably a hard shell capsule.

In an embodiment, the oral pharmaceutical compositions comprise from 1mg to 4mg of pomalidomide.

In an embodiment, the active pharmaceutical ingredient (API) percentage is preferably not more than 5wt.% of the capsule content.

In an embodiment, the method for manufacturing the oral pharmaceutical compositions comprising pomalidomide comprises the following steps:
(a) calibrating, blending and mixing the drug substance, diluents(s), binder(s) and, where applicable, disintegrant(s),
(b) calibrating the lubricant and adding it to the mixture,
(c) lubricating the mixture,
(d) filling the final blend into capsules,
(e) packaging.

In an embodiment, as an example (Example 1 - Pharmaceutical compositions), tables 1 to 4 show the formulations of the pomalidomide pharmaceutical compositions described in the present disclosure.

**Table 1 - Qualitative and quantitative composition data**

| **Ingredient** | **Function** | **Composition 1a** | | **Composition 1b** | |
|---|---|---|---|---|---|
| | | Quantity [mg] | %* | Quantity | %* |
| Pomalidomide | API | 4.00 | 1.43 | 4.00 | 1.43 |
| Anhydrous Lactose | Diluent | 197.60 | 70.57 | 169.60 | 60.57 |
| Microcrystal li ne cellulose | Diluent | 35.00 | 12.50 | 35.00 | 12.50 |
| Hypromellose | Binder | 28.00 | 10.00 | 56.00 | 20.00 |
| Croscamellose sodium | Disintegrant | 14.00 | 5.00 | 14.00 | 5.00 |
| Magnesium Stearate | Lubricant | 1.40 | 0.50 | 1.40 | 0.50 |
| TOTAL | | 280.00 | 100.00 | 280.00 | 100.00 |

| | | | | | |
|---|---|---|---|---|---|
| * Each ingredient is expressed as a percentage (wt/wt) of the total mixture | | | | | |

**Table 2 - Qualitative and quantitative composition data**

| **Ingredient** | **Function** | **Composition 2a** | | **Composition 2b** | | **Composition 2c** | | **Composition 2d** | |
|---|---|---|---|---|---|---|---|---|---|
| | | Quant. [mg] | %* | Quant. [mg] | %* | Quant. [mg] | %* | Quant. [mg] | %* |
| Pomalidomide | API | 4.00 | 1.43 | 4.00 | 1.43 | 4.00 | 1.43 | 4.00 | 1.43 |
| Anhydrous Lactose | Diluent | 211.06 | 75.57 | 183.60 | 65.57 | 232.60 | 83.07 | 204.60 | 73.07 |
| Microcrystalline cellulose | Diluent | 35.00 | 12.50 | 35.00 | 12.50 | - | - | - | - |
| Hypromellose | Binder | 28.00 | 10.00 | 56.00 | 20.00 | 28.00 | 10.00 | 56.00 | 20.00 |
| Croscarmellose sodium | Disintegr ant | - | - | - | - | 14.00 | 5.00 | 14.00 | 5.00 |
| Magnesium Stearate | Lubricant | 1.40 | 0.50 | 1.40 | 0.50 | 1.40 | 0.50 | 1.40 | 0.50 |
| TOTAL | | 280.00 | 100.00 | 280.00 | 100.00 | 280.00 | 100.00 | 280.00 | 100.00 |

| | | | | | | | | | |
|---|---|---|---|---|---|---|---|---|---|
| * Each ingredient is expressed as a percentage (wt/wt) of the total mixture | | | | | | | | | |

**Table 3 - Qualitative and quantitative composition data**

| **Ingredient** | **Function** | **Composition 3a** | | **Composition 3b** | |
|---|---|---|---|---|---|
| | | Quantity [mg] | %* | Quantity | %* |
| Pomalidomide | API | 4.00 | 1.43 | 4.00 | 1.43 |
| Anhydrous Lactose | Diluent | 246.60 | 88.07 | 218.60 | 78.07 |
| Microcrystalli ne cellulose | Diluent | - | - | - | - |
| Hypromellose | Binder | 28.00 | 10.00 | 56.00 | 20.00 |
| Croscamellose sodium | Disintegrant | - | - | - | - |
| Magnesium Stearate | Lubricant | 1.40 | 0.50 | 1.40 | 0.50 |
| TOTAL | | 280.00 | 100.00 | 280.00 | 100.00 |

| | | | | | |
|---|---|---|---|---|---|
| * Each ingredient is expressed as a percentage (wt/wt) of the total mixture | | | | | |

**Table 4- Qualitative and quantitative composition data**

| **Ingredient** | **Function** | **Composition 4a** | | **Composition 4b** | | **Composition 4c** | | **Composition 2a** | |
|---|---|---|---|---|---|---|---|---|---|
| | | Quan t. [mg] | %* | Quant . [mg] | %* | Quant . [mg] | %* | Quant . [mg] | %* |
| Pomalidomide | API | 1.00 | 0.36 | 2.00 | 0.71 | 3.00 | 1.07 | 4.00 | 1.43 |
| Anhydrous Lactose | Diluent | 214.6 0 | 76.64 | 213.60 | 76.29 | 212.60 | 75.93 | 211.60 | 75.57 |
| Microcrystalli ne cellulose | Diluent | 35.00 | 12.50 | 35.00 | 12.50 | 35.00 | 12.50 | 35.00 | 12.50 |
| Hypromellose | Binder | 28.00 | 10.00 | 28.00 | 10.00 | 28.00 | 10.00 | 28.00 | 10.00 |
| Magnesium Stearate | Lubricant | 1.40 | 0.50 | 1.40 | 0.50 | 1.40 | 0.50 | 1.40 | 0.50 |
| TOTAL | | 280.0 0 | 100.00 | 280.00 | 100.00 | 280.00 | 100.00 | 280.00 | 100.00 |

| | | | | | | | | | |
|---|---|---|---|---|---|---|---|---|---|
| * Each ingredient is expressed as a percentage (wt/wt) of the total mixture | | | | | | | | | |

In an embodiment, according to Table 4, the powder mixture mass (280 mg) is the same for compositions 4a, 4b, 4c and 2a. The qualitative composition is the same for 1 mg, 2mg, 3mg and 4 mg compositions. The API percentage ranges from 0.1% to 3% by weight (wt/wt), preferably range from 0.2% to 2% by weight (wt/wt), more preferably range from 0.3% to 1.5% by weight (wt/wt).

In an embodiment, as an example (Example 2 - Dissolution profiles), the dissolution profiles of the example compositions are listed in Table 5.

**Table 5 - Dissolution profiles of compositions**

| **Time (min)** | **Comp 1a** | **Comp 1b** | **Comp 2a** | **Comp 2b** | **Comp 2c** | **Comp 2d** | **Comp 3a** | **Comp 3b** | **Comp 4a** |
|---|---|---|---|---|---|---|---|---|---|
| **0** | 0 | 0 | 0 | 0 | 0 | 0 | 0 | 0 | 0 |
| **5** | 39 | 45 | 48 | 35 | 48 | 36 | 53 | 36 | 54 |
| **10** | 62 | 67 | 74 | 62 | 71 | 66 | 72 | 64 | 78 |
| **15** | 71 | 75 | 81 | 68 | 82 | 78 | 77 | 75 | 87 |
| **20** | 77 | 80 | 85 | 76 | 85 | 84 | 80 | 81 | 90 |
| **30** | 84 | 84 | 88 | 84 | 91 | 89 | 84 | 88 | 93 |
| **45** | 87 | 90 | 91 | 89 | 93 | 93 | 89 | 93 | 97 |
| **60** | 91 | 91 | 93 | 91 | 95 | 95 | 91 | 95 | 98 |
| **90** | 93 | 93 | 94 | 94 | 98 | 97 | 93 | 97 | 99 |

Tables 5 discloses the dissolution profiles of example Composition 1a, Composition 1b, Composition 2a, Composition 2b, Composition 2c, Composition 2d, Composition 3a, Composition 3b, Composition 4a. The example compositions of the present disclosure showed an advantageous immediate release profile with high dose dissolution at the 60 minutes time point. All example compositions comprising pomalidomide, anhydrous lactose and hypromellose have an advantageous immediate release profile with high dose dissolution at the 60 minutes time point.

In an embodiment, as an example (Example 3 - Comparative pharmaceutical formulations), Tables 6 to 8 show the composition data of comparative formulations. These comparative formulations are to be compared against the example compositions of the present disclosure.

**Table 6 - Qualitative and quantitative comparative compositions**

| **Ingredient** | **Function** | **Composition 5a** | | **Composition 5b** | |
|---|---|---|---|---|---|
| | | Quantity [mg] | %* | Quantity | %* |
| Pomalidomide | API | 4.00 | 1.43 | 4.00 | 1.43 |
| Anhydrous Lactose | Diluent | 218.60 | 78.07 | 246.60 | 88.07 |
| Microcrystalli ne cellulose | Diluent | - | - | - | - |
| Hydroxypropyl cellulose | Binder | 56.00 | 20.00 | - | - |
| Crospovidone | Binder | - | - | 28.00 | 10.00 |
| Croscamellose sodium | Disintegrant | - | - | - | - |
| Magnesium Stearate | Lubricant | 1.40 | 0.50 | 1.40 | 0.50 |
| TOTAL | | 280.00 | 100.00 | 280.00 | 100.00 |

| | | | | | |
|---|---|---|---|---|---|
| * Each ingredient is expressed as a percentage (wt/wt) of the total mixture | | | | | |

**Table 7 - Qualitative and quantitative comparative compositions**

| **Ingredient** | **Function** | **Composition 6a** | | **Composition 6b** | | **Composition 6c** | | **Composition 6d** | |
|---|---|---|---|---|---|---|---|---|---|
| | | Quant . [mg] | %* | Quant. [mg] | %* | Quant. [mg] | %* | Quant. [mg] | %* |
| Pomalidomide | API | 4.00 | 1.43 | 4.00 | 1.43 | 4.00 | 1.43 | 4.00 | 1.43 |
| Anhydrous Lactose | Diluent | 211.6 0 | 75.57 | 222.80 | 79.57 | 211.60 | 75.57 | 183.60 | 65.57 |
| Microcrystalline cellulose | Diluent | 35.00 | 12.50 | 35.00 | 12.50 | 35.00 | 12.50 | 35.00 | 12.50 |
| Hydroxypropyl cellulose | Binder | 28.00 | 10.00 | - | - | - | - | - | - |
| Crospovidone | Binder | - | - | 16.80 | 6.00 | 28.00 | 10.00 | 56.00 | 20.00 |
| Croscarmellose sodium | Disintegrant | - | - | - | - | - | - | - | - |
| Magnesium Stearate | Lubricant | 1.40 | 0.50 | 1.40 | 0.50 | 1.40 | 0.50 | 1.40 | 0.50 |
| TOTAL | | 280.0 | 1000 0 | 280.00 | 100.00 | 280.00 | 100.00 | 280.00 | 100.00 |

| | | | | | | | | | |
|---|---|---|---|---|---|---|---|---|---|
| * Each ingredient is expressed as a percentage (wt/wt) of the total mixture | | | | | | | | | |

**Table 8 - Qualitative and quantitative comparative compositions**

| **Ingredient** | **Function** | **Composition 7a** | | **Composition 7b** | |
|---|---|---|---|---|---|
| | | Quant. [mg] | %* | Quant.[mg] | %* |
| Pomalidomide | API | 4.01 | 1.67 | 4.01 | 1.67 |
| Anhydrous Lactose | Diluent | 204.79 | 85.30 | 234.79 | 97.83 |
| Microcrystalline cellulose | Diluent | 30.00 | 12.50 | - | - |
| Croscarmellose sodium | Disintegrant | - | - | - | - |
| Magnesium Stearate | Lubricant | 1.20 | 0.50 | 1.20 | 0.50 |
| TOTAL | | 240.00 | 100.00 | 240.00 | 100.00 |

| | | | | | |
|---|---|---|---|---|---|
| * Each ingredient is expressed as a percentage (wt/wt) of the total mixture | | | | | |

In an embodiment, as an example (Example 4 - Dissolution profiles of comparative compositions), the dissolutions profiles of the comparative compositions are listed in Table 9.

**Table 9 - Dissolution profiles of comparative compositions**

| **Time (min)** | **Compar. Comp. 5a** | **Compar. Comp. 5b** | **Compar. Comp. 6a** | **Compar. Comp. 6b** | **Compar. Comp. 6c** | **Compar. Comp. 6d** | **Compar. Comp. 7a** | **Compar. Comp. 7b** |
|---|---|---|---|---|---|---|---|---|
| **0** | 0 | 0 | 0 | 0 | 0 | 0 | 0 | 0 |
| **5** | 39 | 42 | 37 | 31 | 35 | 34 | 44 | 37 |
| **10** | 51 | 60 | 59 | 56 | 55 | 54 | 63 | 58 |
| **15** | 64 | 68 | 70 | 65 | 60 | 62 | 72 | 65 |
| **20** | 68 | 74 | 76 | 71 | 66 | 67 | 76 | 70 |
| **30** | 75 | 80 | 82 | 77 | 72 | 75 | 82 | 76 |
| **45** | 81 | 83 | 87 | 84 | 77 | 82 | 86 | 80 |
| **60** | 84 | 89 | 89 | 87 | 81 | 86 | 88 | 82 |
| **90** | 89 | 91 | 90 | 91 | 87 | 91 | 90 | 84 |

Tables 9 discloses the dissolution profiles of Comparative Composition 5a, Comparative Composition 5b, Comparative Composition 6a, Comparative Composition 6b, Comparative Composition 6c, Comparative Composition 6d, Comparative Composition 7a and Comparative Composition 7b. As compared to the example compositions, the comparative compositions do not have an advantageous immediate release profile with high dose dissolution at the 60 minutes time point.

In an embodiment, as an example (Example 5 - Stability testing of the formulations), the stability of the example compositions was analyzed.

Example Composition 1a, Composition 2a, Composition 2c, Composition 2d, Composition 3b and Composition 4a were submitted to stability studies at several standard conditions. The results obtained are presented in Tables 10 to 18.

**Table 10 - Assay and related compounds stability results - Composition 1a**

| **Compound** | **Specification** | **T=0** | **1 Month** | **3 Months** | | |
|---|---|---|---|---|---|---|
| | | | 30°C/75RH Open flask | 40°C/60RH OPA blister | 30°/75RH OPA blister | 25°C/60RH OPA blister |
| **POMA-03** | **< 1.0%** | 0.09 | 0.09 | 0.09 | 0.09 | 0.09 |
| **Thalidomi de** | **< 1.0%** | 0.03 | 0.02 | 0.03 | 0.02 | 0.03 |
| **POMA 4-Isomer** | **< 1.0%** | 0.03 | 0.01 | 0.02 | 0.03 | 0.020 |
| **POMA-OH** | **< 1.0%** | 0.05 | 0.04 | 0.05 | 0.05 | 0.05 |
| **S.L.U. Impurity** | **< 0.5%** | 0.04 | 0.05 | 0.04 | 0.04 | 0.04 |
| **TOTAL Imp.** | **< 5.0%** | 0.38 | 0.30 | 0.30 | 0.31 | 0.30 |
| **Assay** | **95.0%-105.0%** | 99.7 | 102.8 | 100.8 | 100.5 | 99.7 |

**Table 11- Assay and related compounds stability results - Composition 2a**

| **Compou nd** | **Speci ficatio n** | **T=0** | **1 Month** | | **2 Months** | | | **3 Months** | | |
|---|---|---|---|---|---|---|---|---|---|---|
| | | | 25°C/ 60RH Open flask | 40°C/75 RH Open flask | 25°C /60R H Open flask | 40°C/75R H Open flask | 30°C/75 RH OPA blister | 25°C/60R H OPA blister | 30°C/75R H OPA blister | 40°C/75R H OPA blister |
| **POMA-03** | **< 1.0%** | 0.08 | 0.09 | 0.09 | 0.09 | 0.09 | 0.09 | 0.08 | 0.10 | 0.09 |
| **Thalido mide** | **< 1.0%** | 0.02 | 0.02 | 0.02 | 0.02 | 0.02 | 0.02 | 0.02 | 0.03 | 0.03 |
| **POMA 4-Isomer** | **< 1.0%** | 0.02 | 0.04 | 0.02 | 0.02 | 0.02 | 0.02 | 0.02 | 0.04 | 0.03 |
| **POMA-OH** | **< 1.0%** | 0.04 | 0.05 | 0.05 | 0.04 | 0.04 | 0.05 | 0.04 | 0.05 | 0.04 |
| **S.L.U. Impurity** | **< 0.5%** | 0.05 | 0.05 | 0.03 | 0.04 | 0.04 | 0.05 | 0.05 | 0.05 | 0.05 |
| **TOTAL Imp.** | **< 5.0%** | 0.34 | 0.41 | 0.32 | 0.35 | 0.34 | 0.41 | 0.33 | 0.46 | 0.38 |
| **Assay** | **95.0 %-105.0 %** | 99.6 | 99.6 | 99.7 | 99.6 | 101.4 | 99.9 | 100.6 | 100.5 | 100.8 |

**Table 12 - Assay and related compounds stability results - Composition 2a (continued)**

| **Compou nd** | **Speci ficatio n** | **3 Months** | | | **6 Months** | | | | | |
|---|---|---|---|---|---|---|---|---|---|---|
| | | 40°C/75 RH PVC blister | 30°C/75 RH PVC blister | 25°C/60 RH PVC blister | 40°C/75R H OPA blister | 40°C/75R H PVC blister | 30°C/75 RH OPA blister | 30°C/75R H PVC blister | 25°C/60 RH OPA blister | 25°C/60R H PVC blister |
| **POMA-03** | < **1.0%** | 0.08 | 0.09 | 0.08 | 0.09 | 0.09 | 0.09 | 0.09 | 0.09 | 0.09 |
| **Thalido mide** | **< 1.0%** | 0.03 | 0.04 | 0.03 | 0.02 | 0.03 | 0.02 | 0.03 | 0.02 | 0.03 |
| **POMA 4-Isomer** | **< 1.0%** | 0.02 | 0.22 | 0.01 | 0.02 | 0.02 | 0.02 | 0.02 | 0.02 | 0.02 |
| **POMA-OH** | **< 1.0%** | 0.04 | 0.04 | 0.04 | 0.05 | 0.02 | 0.05 | 0.05 | 0.05 | 0.05 |
| **S.L.U. Impurity** | **< 0.5%** | 0.05 | 0.04 | 0.05 | 0.04 | 0.04 | 0.04 | 0.04 | 0.04 | 0.04 |
| **TOTAL Imp.** | **< 5.0%** | 0.34 | 0.38 | 0.30 | 0.29 | 0.29 | 0.29 | 0.30 | 0.29 | 0.29 |
| **Assay** | **95.0 %-105.0 %** | 100.2 0 | 99.0 | 98.7 | 98.8 | 97.0 | 99.1 | 99.1 | 99.2 | 98.5 |

**Table 13 - Assay and related compounds stability results - Composition 2c**

| **Compound** | **Specification** | **T=0** | **1 Month** | **3 Months** | | |
|---|---|---|---|---|---|---|
| | | | 30°C/75RH Open flask | 40°C/75RH OPA blister | 30°/75RH OPA blister | 25°C/60RH OPA blister |
| **POMA-03** | **< 1.0%** | 0.09 | 0.09 | 0.09 | 0.09 | 0.09 |
| **Thalidomi de** | **< 1.0%** | 0.03 | 0.03 | 0.03 | 0.03 | 0.03 |
| **POMA 4-Isomer** | **< 1.0%** | 0.03 | 0.03 | 0.02 | 0.02 | 0.02 |
| **POMA-OH** | **< 1.0%** | 0.05 | 0.05 | 0.05 | 0.05 | 0.05 |
| **S.L.U. Impurity** | **< 0.5%** | 0.04 | 0.04 | 0.04 | 0.04 | 0.04 |
| **TOTAL Imp.** | < **5.0%** | 0.38 | 0.37 | 0.31 | 0.31 | 0.31 |
| **Assay** | **95.0%-105.0%** | 99.4 | 100.2 | 99.6 | 100.8 | 99.9 |

**Table 14 - Assay and related compounds stability results - Composition 2d**

| **Compou nd** | **Specification** | **T=0** | **1 Month** | | **2 Months** | | |
|---|---|---|---|---|---|---|---|
| | | | 40°C/75RH Open flask | 25°C/60RH Open flask | 40°C/75RH Open flask | 25°/60RH Open flask | 30°C/75RH OPA blister |
| **POMA-03** | < **1.0%** | 0.08 | 0.09 | 0.09 | 0.09 | 0.09 | 0.09 |
| **Thalido mide** | < **1.0%** | 0.02 | 0.02 | 0.02 | 0.02 | 0.02 | 0.02 |
| **POMA 4-Isomer** | < **1.0%** | 0.02 | 0.02 | 0.02 | 0.02 | 0.02 | 0.02 |
| **POMA-OH** | < **1.0%** | 0.04 | 0.05 | 0.05 | 0.04 | 0.06 | 0.05 |
| **S.L.U. Impurity** | **< 0.5%** | 0.04 | 0.04 | 0.05 | 0.04 | 0.25 | 0.04 |
| **TOTAL Imp.** | < **5.0%** | 0.4 | 0.38 | 0.39 | 0.32 | 0.64 | 0.36 |
| **Assay** | **95.0%-105.0%** | 102.3 | 100.1 | 100.3 | 103.2 | 101.1 | 103.0 |

**Table 15 - Assay and related compounds stability results - Composition 2d (continued)**

| **Compou nd** | **Specification** | **3 Months** | | | | | |
|---|---|---|---|---|---|---|---|
| | | 40°C/75RH OPA blister | 40°C/75RH PVC blister | 30°C/75RH OPA blister | 30°C/75RH PVC blister | 25°/60RH OPA blister | 25°C/60RH PVC blister |
| **POMA-03** | **< 1.0%** | 0.09 | 0.09 | 0.09 | 0.09 | 0.09 | 0.09 |
| **Thalido mide** | **< 1.0%** | 0.02 | 0.03 | 0.03 | 0.03 | 0.02 | 0.02 |
| **POMA 4-Isomer** | **< 1.0%** | 0.02 | 0.03 | 0.03 | 0.02 | 0.02 | 0.02 |
| **POMA-OH** | **< 1.0%** | 0.04 | 0.05 | 0.05 | 0.04 | 0.04 | 0.04 |
| **S.L.U. Impurity** | **< 0.5 %** | 0.05 | 0.05 | 0.04 | 0.05 | 0.05 | 0.05 |
| **TOTAL Imp.** | **< 5.0%** | 0.33 | 0.39 | 0.45 | 0.30 | 0.33 | 0.32 |
| **Assay** | **95-105** | 100.9 | 102.52 | 100.78 | 99.62 | 101.33 | 100.99 |

**Table 16 - Assay and related compounds stability results - Composition 3b**

| **Compou nd** | **Specification** | **T=0** | **1 Month** | | **2 Months** | | |
|---|---|---|---|---|---|---|---|
| | | | 40°C/75RH Open flask | 25°C/60RH Open flask | 40°C/75RH Open flask | 25°/60RH Open flask | 30°C/75RH OPA blister |
| **POMA-03** | **< 1.0%** | 0.08 | 0.09 | 0.09 | 0.09 | 0.09 | 0.09 |
| **Thalido mide** | **< 1.0%** | 0.03 | 0.02 | 0.02 | 0.02 | 0.02 | 0.02 |
| **POMA 4-Isomer** | **< 1.0%** | 0.02 | 0.02 | 0.02 | 0.02 | 0.02 | 0.02 |
| **POMA-OH** | **< 1.0%** | 0.04 | 0.04 | 0.05 | 0.04 | 0.05 | 0.05 |
| **S.L.U. Impurity** | **< 0.5%** | 0.05 | 0.04 | 0.06 | 0.04 | 0.24 | 0.7 |
| **TOTAL Imp.** | **< 5.0%** | 0.36 | 0.37 | 0.44 | 0.34 | 0.61 | 0.44 |
| **Assay** | **95.0%-105.0%** | 102.3 | 98.4 | 99.3 | 102.4 | 99.5 | 100.6 |

**Table 17 - Assay and related compounds stability results - Composition 3b (continued)**

| **Compou nd** | **Specification** | **3 Months** | | | | | |
|---|---|---|---|---|---|---|---|
| | | 40°C/75RH OPA blister | 40°C/75RH PVC blister | 30°C/75RH OPA blister | 30°C/75RH PVC blister | 25°/60RH OPA blister | 25°C/60RH PVC blister |
| **POMA-03** | **< 1.0%** | 0.09 | 0.08 | 0.10 | 0.10 | 0.09 | 0.09 |
| **Thalido mide** | **< 1.0%** | 0.03 | 0.03 | 0.03 | 0.05 | 0.02 | 0.02 |
| **POMA 4-Isomer** | **< 1.0%** | 0.02 | 0.02 | 0.04 | 0.04 | 0.01 | 0.02 |
| **POMA-OH** | **< 1.0%** | 0.04 | 0.04 | 0.05 | 0.05 | 0.04 | 0.05 |
| **S.L.U. Impurity** | **< 0.5%** | 0.04 | 0.04 | 0.05 | 0.05 | 0.05 | 0.05 |
| **TOTAL Imp.** | **< 5.0%** | 0.32 | 0.35 | 0.47 | 0.50 | 0.30 | 0.34 |
| **Assay** | **95.0%-105.0%** | 99.8 | 99.4 | 100.3 | 100.6 | 100.7 | 100.3 |

**Table 18 - Assay and related compounds stability results - Composition 4a**

| **Compo und** | **Speci ficatio n** | **T=0** | **1 Month** | | **2 Months** | | | **3 Months** | | | |
|---|---|---|---|---|---|---|---|---|---|---|---|
| | | | 25°C /60R H Open flask | 40°C/75 RH Open flask | 25°C /60R H Open flask | 40°C/75 RH Open flask | 30°C/75 RH OPA blister | 25°C/60 RH OPA blister | 30°C/75 RH OPA blister | 40°C/75 RH* OPA blister | 40°C/75 RH PVC/PC TFE blister |
| **POMA-03** | **< 1.0%** | 0.09 | 0.10 | 0.10 | 0.10 | 0.10 | 0.10 | 0.11 | 0.09 | 0.10 | 0.09 |
| **Thalid omide** | **< 1.0%** | 0.03 | 0.03 | 0.03 | 0.03 | 0.02 | 0.02 | 0.02 | 0.03 | 0.03 | 0.03 |
| **POMA 4-Isomer** | **< 1.0%** | 0.02 | 0.03 | 0.03 | 0.02 | 0.02 | 0.02 | 0.05 | 0.03 | 0.03 | 0.03 |
| **POMA-OH** | **< 1.0%** | 0.05 | 0.05 | 0.05 | 0.05 | 0.05 | 0.05 | 0.06 | 0.04 | 0.05 | 0.05 |
| **S.L.U. Impuri ty** | **< 0.5%** | 0.06 | 0.07 | 0.07 | 0.06 | 0.08 | 0.05 | 0.07 | 0.05 | 0.11 | 0.10 |
| **TOTAL Imp.** | **< 5.0%** | 0.54 | 0.49 | 0.49 | 0.46 | 0.48 | 0.43 | 0.54 | 0.41 | 0.51 | 0.49 |
| **Assay** | **95.0 %-105.0 %** | 102.9 | 102.2 | 101.7 | 102.2 | 103.4 | 103.3 | 103.0 | 103.4 | 103.6 | 102.2 |

The controlled related compounds referred to in Tables 10 to 18 are: POMA03 (Nitropomalidomide) CAS 19171-18-7; Thalidomide CAS 50-35-1; POMA4-isomer CAS 191732-76-0; POMA-OH CAS 1547162-41-3 and S.L.U. Impurity refers to the Single Largest Unknown Impurity detected.

The assay and related compounds tests results in Tables 10 to 18 show that example Composition 1a, Composition 2a, Composition 2c, Composition 2d, Composition 3b and Composition 4a are stable despite being submitted to various standard conditions (accelerated and long term conditions). The example compositions comprising pomalidomide, anhydrous lactose and hypromellose are stable despite being submitted to various standard conditions (accelerated and long term conditions).

In an embodiment, the formulation according to the disclosure meet the specifications for all the packaging materials that were investigated, in particular OPA blisters.

In an embodiment, as an example (Example 6 - In-vitro dissolution testing of stability batches), the dissolution profiles of several example compositions.

(Composition 2c, Composition 2d, Composition 3b and Composition 4a) were analysed after the example compositions were subjected to stability studies. The results obtained are shown in Tables 19 to 22.

**Table 19 - In-vitro dissolution data of stability batches - Composition 2c**

| **Time (min)** | **T=0** | **25°C/60%RH** | | | **30°C/75%RH** | | |
|---|---|---|---|---|---|---|---|
| | | **2 Months OPA blister** | **3 Months OPA blister** | **4 Months OPA blister** | **1 Month Open flask** | **3 Months OPA blister** | **4 Months OPA blister** |
| **0** | 0 | 0 | 0 | 0 | 0 | 0 | 0 |
| **5** | 48 | 46 | 49 | 43 | 46 | 41 | 42 |
| **10** | 71 | 67 | 76 | 64 | 68 | 66 | 63 |
| **15** | 82 | 73 | 82 | 70 | 76 | 77 | 71 |
| **20** | 85 | 77 | 86 | 77 | 80 | 83 | 76 |
| **30** | 91 | 83 | 90 | 83 | 84 | 87 | 85 |
| **45** | 93 | 88 | 93 | 88 | 88 | 91 | 89 |
| **60** | 95 | 92 | 95 | 91 | 90 | 94 | 92 |
| **90** | 98 | 94 | 97 | 94 | 92 | 96 | 94 |

**Table 20 - In-vitro dissolution data of stability batches - Composition 2d**

| **Time (min)** | **T=0** | **25°C/60%RH** | | | **30°C/75%RH** | | |
|---|---|---|---|---|---|---|---|
| | | **1 Month Open flask** | **3 Months OPA blister** | **4 Months OPA blister** | **1 Month OPA blister** | **2 Months OPA blister** | **3 Months OPA blister** |
| **0** | 0 | 0 | 0 | 0 | 0 | 0 | 0 |
| **5** | 36 | 40 | 39 | 29 | 31 | 28 | 20 |
| **10** | 66 | 65 | 63 | 54 | 51 | 52 | 45 |
| **15** | 78 | 75 | 77 | 65 | 67 | 66 | 57 |
| **20** | 84 | 82 | 83 | 72 | 78 | 75 | 69 |
| **30** | 89 | 86 | 91 | 81 | 85 | 87 | 82 |
| **45** | 93 | 90 | 94 | 87 | 90 | 91 | 88 |
| **60** | 95 | 92 | 95 | 91 | 92 | 93 | 91 |
| **90** | 97 | 94 | 97 | 95 | 94 | 96 | 94 |

**Table 21 - In-vitro dissolution data of stability batches - Composition 3b**

| **Time (min)** | **T=0** | **25°C/60%RH** | | | **30°C/75%RH** | | |
|---|---|---|---|---|---|---|---|
| | | **1 Month Open flask** | **3 Months OPA blister** | **4 Months OPA blister** | **1 Month OPA blister** | **2 Months OPA blister** | **3 Months OPA blister** |
| **0** | 0 | 0 | 0 | 0 | 0 | 0 | 0 |
| **5** | 36 | 28 | 33 | 29 | 30 | 22 | 21 |
| **10** | 64 | 52 | 59 | 53 | 58 | 54 | 48 |
| **15** | 75 | 68 | 72 | 67 | 69 | 66 | 63 |
| **20** | 81 | 78 | 80 | 74 | 74 | 75 | 72 |
| **30** | 88 | 86 | 88 | 81 | 81 | 84 | 83 |
| **45** | 93 | 88 | 92 | 87 | 86 | 90 | 90 |
| **60** | 95 | 90 | 92 | 90 | 90 | 92 | 92 |
| **90** | 97 | 93 | 94 | 93 | 92 | 95 | 94 |

**Table 22- In-vitro dissolution data of stability batches- Composition 4a**

| **Time (min)** | **T=0** | **25°C/60%RH** | | | | | **25°C/60%RH** | | | | | |
|---|---|---|---|---|---|---|---|---|---|---|---|---|
| | | **1 Month Open flask** | **3 Month s OPA blister** | **4 Month s OPA blister** | **5 Month s OPA blister** | **6 Month sOPA blister** | **1 Month OPA blister** | **2 Month s OPA blister** | **3 Month s OPA blister** | **4 Month s OPA blister** | **5 Month s OPA blister** | **6 Month s OPA blister** |
| **0** | 0 | 0 | 0 | 0 | 0 | 0 | 0 | 0 | 0 | 0 | 0 | 0 |
| **5** | 54 | 43 | 57 | 49 | 44 | 45 | 53 | 50 | 49 | 52 | 45 | 51 |
| **10** | 78 | 67 | 74 | 70 | 68 | 68 | 73 | 71 | 69 | 73 | 69 | 75 |
| **15** | 87 | 77 | 80 | 79 | 79 | 80 | 81 | 79 | 79 | 82 | 78 | 82 |
| **20** | 90 | 81 | 84 | 87 | 83 | 86 | 86 | 83 | 82 | 85 | 83 | 87 |
| **30** | 93 | 87 | 90 | 90 | 89 | 92 | 89 | 90 | 87 | 92 | 88 | 92 |
| **45** | 97 | 90 | 92 | 93 | 91 | 94 | 93 | 92 | 90 | 94 | 92 | 94 |
| **60** | 98 | 91 | 93 | 95 | 93 | 95 | 94 | 94 | 91 | 94 | 92 | 95 |
| **90** | 99 | 92 | 94 | 96 | 94 | 96 | 97 | 95 | 93 | 96 | 94 | 96 |

The dissolution profiles disclosed in Tables 19 to 22 show that the example compositions maintained their advantageous immediate release profile with high dose dissolution at the 60 minutes time point even after being submitted to stability conditions (accelerated and long term conditions).

In an embodiment, *in vitro* dissolution data (table 5, table 9 and tables 19 to 22) was obtained according to the settings as set out in Table 23.

**Table 23 - in-vitro dissolution settings**

| **Method** | *USP opp. II - Method 2* (*paddle, spirale sinker*) |
|---|---|
| **Medium** | HCl 0.1N |
| **Volume** | *900ml* |
| **Temperature** | *37.0°C ± 0.5°C* |
| **Stirring speed** | *50 rpm* |

The term "comprising" whenever used in this document is intended to indicate the presence of stated features, integers, steps, components, but not to preclude the presence or addition of one or more otherfeatures, integers, steps, components or groups thereof.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

The disclosure should not be seen in any way restricted to the embodiments described and a person with ordinary skill in the art will foresee many possibilities to modifications thereof. The above described embodiments are combinable.

## Claims

1. An immediate release pharmaceutical composition comprising
0.10 % to 3.00 % (wt/wt) of pomalidomide;
40.00 % to 93.00 % (wt/wt) of anhydrous lactose as a diluent;
6.00 % to 25.00 % (wt/wt) of hypromellose as a binder.

2. The pharmaceutical composition according to claim 1 wherein the amount of pomalidomide is from 0.2% to 2% (wt/wt), preferably from 0.3% to 1.5% (wt/wt).

3. The pharmaceutical composition according to any of the previous claims wherein the amount of anhydrous lactose ranges from 50% to 90% (wt/wt).

4. The pharmaceutical composition according to any of the previous claims wherein the amount of hypromellose ranges from 8% to 22% (wt/wt).

5. The pharmaceutical composition according to any of the previous claims further comprising 0.2% to 2% (wt/wt) of lubricant.

6. The pharmaceutical composition according to claim 5 wherein the lubricant is selected from: magnesium stearate, sodium stearyl fumarate, calcium stearate, stearic acid,
glyceryl behenate, hexanedioic acid, hydrogenated vegetable oil, glycerine fumarate, or mixtures thereof.

7. The pharmaceutical composition according to any of the previous claims further comprising one or more excipients selected from
2% to 10% (wt /wt) of a disintegrant and/or;
5% to 20% (wt/wt), preferably 10% to 20% (wt/wt), more preferably 10% to 15% (wt/wt) of at least a further diluent.

8. The pharmaceutical composition according to the previous claim wherein the disintegrant is selected from: sodium croscarmellose, sodium carboxymethyl cellulose, sodium starch glycolate, crospovidone, or mixtures thereof.

9. The pharmaceutical composition according to any of the previous claims 7-8 wherein the further diluent is microcrystalline cellulose.

10. The pharmaceutical composition according to any of the previous claims comprising:
1.43% (wt/wt) pomalidomide;
75.57 % (wt/wt) anhydrous lactose;
12.50 % (wt/wt) microcrystalline cellulose;
10.00 % (wt/wt) hypromellose;
0.50 % (wt/wt) magnesium stearate.

11. The pharmaceutical composition according to any of the previous claims 1-9 comprising:
0.36 % (wt/wt) pomalidomide;
76.64 % (wt/wt) anhydrous lactose;
12.50 % (wt/wt) microcrystalline cellulose;
10.00 % (wt/wt) hypromellose;
0.50 % (wt/wt) magnesium stearate.

12. The pharmaceutical composition according to any of the previous claims 1-8 comprising:
1.43 % (wt/wt) pomalidomide;
83.07 % (wt/wt) anhydrous lactose;
5.00 % (wt/wt) sodium croscarmellose;
10.00 % (wt/wt) hypromellose;
0.50 % (wt/wt) magnesium stearate.

13. The pharmaceutical composition according to any of the previous claims wherein the pomalidomide has a particle size **characterized by** a D50 ranging from 3µm to 20µm, preferably ranging from 5µm to 10µm.

14. The pharmaceutical composition according to any of the previous claims wherein the composition is a capsule, preferably a hard shell capsule.

15. The pharmaceutical composition according to any of the previous claims comprising 1 mg to 4 mg of pomalidomide.

16. The pharmaceutical composition according to any of the previous claims wherein the pharmaceutical ingredient percentage is not more than 5.00 wt.% of total capsule content.

17. The pharmaceutical composition according to any of the previous claims for use in the treatment of cancer, preferably multiple myeloma.

## Patentansprüche

1. Eine pharmazeutische Zusammensetzung mit sofortiger Freisetzung, umfassend
0,10 % bis 3,00 % (Gew./Gew.) Pomalidomid;
40,00 % bis 93,00 % (Gew./Gew.) wasserfreie Laktose als Verdünnungsmittel;
6,00 % bis 25,00 % (Gew./Gew.) Hypromellose als Bindemittel.

2. Die pharmazeutische Zusammensetzung nach Anspruch 1, wobei die Menge an Pomalidomid 0,2 % bis 2 % (Gew./Gew.), bevorzugt 0,3 % bis 1,5 % (Gew./Gew.) beträgt.

3. Die pharmazeutische Zusammensetzung nach einem der vorangehenden Ansprüche, wobei die Menge an wasserfreier Laktose im Bereich von 50 % bis 90 % (Gew./Gew.) liegt.

4. Die pharmazeutische Zusammensetzung nach einem der vorangehenden Ansprüche, wobei die Menge an Hypromellose im Bereich von 8 % bis 22 % (Gew./Gew.) liegt.

5. Die pharmazeutische Zusammensetzung nach einem der vorangehenden Ansprüche ferner umfassend 0,2 % bis 2 % (Gew./Gew.) eines Gleitmittels.

6. Die pharmazeutische Zusammensetzung nach Anspruch 5, wobei das Gleitmittel ausgewählt wird aus: Magnesiumstearat, Natriumstearylfumarat, Calciumstearat, Stearinsäure, Glycerylbehenat, Hexandisäure, hydriertem Pflanzenöl, Glycerinfumarat oder Mischungen davon.

7. Die pharmazeutische Zusammensetzung nach einem der vorangehenden Ansprüche ferner umfassend einen oder mehrere Hilfsstoffe, ausgewählt aus
2 % bis 10 % (Gew./Gew.) eines Sprengmittels und/oder;
5% bis 20 % (Gew./Gew.), bevorzugt 10 % bis 20 % (Gew./Gew.), besonders bevorzugt
10 % bis 15 % (Gew./Gew.) mindestens eines weiteren Verdünnungsmittels.

8. Die pharmazeutische Zusammensetzung nach dem vorangehenden Anspruch, wobei das Sprengmittel ausgewählt wird aus: Croscarmellose-Natrium, Natriumcarboxymethylcellulose, Natriumstärkeglycolat, Crospovidon oder Mischungen davon.

9. Die pharmazeutische Zusammensetzung nach einem der vorangehenden Ansprüche 7-8, wobei das weitere Verdünnungsmittel mikrokristalline Cellulose ist.

10. Die pharmazeutische Zusammensetzung nach einem der vorangehenden Ansprüche umfassend:
1,43 % (Gew./Gew.) Pomalidomid;
75,57 % (Gew./Gew.) wasserfreie Laktose;
12,50 % (Gew./Gew.) mikrokristalline Cellulose;
10,00 % (Gew./Gew.) Hypromellose;
0,50 % (Gew./Gew.) Magnesiumstearat.

11. Die pharmazeutische Zusammensetzung nach einem der vorangehenden Ansprüche 1-9 umfassend:
0,36 % (Gew./Gew.) Pomalidomid;
76,64 % (Gew./Gew.) wasserfreie Laktose;
12,50 % (Gew./Gew.) mikrokristalline Cellulose;
10,00 % (Gew./Gew.) Hypromellose;
0,50 % (Gew./Gew.) Magnesiumstearat.

12. Die pharmazeutische Zusammensetzung nach einem der vorangehenden Ansprüche 1-8 umfassend:
1,43 % (Gew./Gew.) Pomalidomid;
83,07 % (Gew./Gew.) wasserfreie Laktose;
5,00 % (Gew./Gew.) Croscarmellose-Natrium;
10,00 % (Gew./Gew.) Hypromellose;
0,50 % (Gew./Gew.) Magnesiumstearat.

13. Die pharmazeutische Zusammensetzung nach einem der vorangehenden Ansprüche, wobei das Pomalidomid eine Teilchengröße aufweist, die durch einen D50 im Bereich von 3 µm bis 20 µm, bevorzugt im Bereich von 5 µm bis 10 µm, gekennzeichnet ist.

14. Die pharmazeutische Zusammensetzung nach einem der vorangehenden Ansprüche, wobei die Zusammensetzung eine Kapsel, bevorzugt eine Hartkapsel, ist.

15. Die pharmazeutische Zusammensetzung nach einem der vorangehenden Ansprüche umfassend 1 mg bis 4 mg Pomalidomid.

16. Die pharmazeutische Zusammensetzung nach einem der vorangehenden Ansprüche, wobei der prozentuale Anteil des pharmazeutischen Inhaltsstoffs nicht mehr als 5,00 Gew.-% des gesamten Kapselinhalts beträgt.

17. Die pharmazeutische Zusammensetzung nach einem der vorangehenden Ansprüche zur Verwendung bei der Behandlung von Krebs, bevorzugt eines multiplen Myelom.

## Revendications

1. Une composition pharmaceutique à libération immédiate comprenant :
0,10% à 3,00% (poids/poids) de pomalidomide ;
40,00% à 93,00% (poids/poids) de lactose anhydre en tant que diluant;
6,00% à 25,00% (poids/poids) d'hypromellose en tant que liant.

2. La composition pharmaceutique selon la revendication 1 dans laquelle la quantité de pomalidomide est de 0,2% à 2% (poids/poids), préférablement de 0,3% à 1,5% (poids/poids).

3. La composition pharmaceutique selon l'une quelconque des revendications précédentes dans laquelle la quantité de lactose anhydre varie de 50% à 90% (poids/poids).

4. La composition pharmaceutique selon l'une quelconque des revendications précédentes dans laquelle la quantité d'hypromellose varie de 8% à 22% (poids/poids).

5. La composition pharmaceutique selon l'une quelconque des revendications précédentes comprenant également 0,2% à 2% (poids/poids) de lubrifiant.

6. La composition pharmaceutique selon la revendication 5 dans laquelle le lubrifiant est choisi parmi : du stéarate de magnésium, du stéarylfumarate de sodium, du stéarate de calcium, de l'acide stéarique, du béhénate de glycéryle, de l'acide hexanedioïque, de l'huile végétale hydrogénée, du fumarate de glycérine, ou des mélanges de ceux-ci.

7. La composition pharmaceutique selon l'une quelconque des revendications précédentes comprenant également un ou plusieurs excipients choisis parmi :
2% à 10% (poids/poids) d'un désintégrant et/ou ;
5% à 20% (poids/poids), préférablement 10% à 20% (poids/poids), plus préférablement 10% à 15% (poids/poids) d'au moins un autre diluant.

8. La composition pharmaceutique selon la revendication précédente dans laquelle le désintégrant est choisi parmi : de la croscarmellose sodique, de la carboxyméthylcellulose sodique, du glycolate sodique d'amidon, de la crospovidone, ou des mélanges de ceux-ci.

9. La composition pharmaceutique selon l'une quelconque des revendications 7-8 dans laquelle l'autre diluant est de la cellulose microcristalline.

10. La composition pharmaceutique selon l'une quelconque des revendications précédentes comprenant :
1,43% (poids/poids) de pomalidomide ;
75,57% (poids/poids) de lactose anhydre ;
12,50% (poids/poids) de cellulose microcristalline;
10,00% (poids/poids) d'hypromellose ;
0,50% (poids/poids) de stéarate de magnésium.

11. La composition pharmaceutique selon l'une quelconque des revendications précédentes 1-9 comprenant :
0,36% (poids/poids) de pomalidomide ;
76,64% (poids/poids) de lactose anhydre ;
12,50% (poids/poids) de cellulose microcristalline;
10,00% (poids/poids) d'hypromellose ;
0,50% (poids/poids) de stéarate de magnésium.

12. La composition pharmaceutique selon l'une quelconque des revendications précédentes 1-8 comprenant :
1,43% (poids/poids) de pomalidomide ;
83,07% (poids/poids) de lactose anhydre ;
5,00% (poids/poids) de croscarmellose sodique ;
10,00% (poids/poids) d'hypromellose ;
0,50% (poids/poids) de stéarate de magnésium.

13. La composition pharmaceutique selon l'une quelconque des revendications précédentes dans laquelle le pomalidomide a une taille de particule **caractérisée par** un D50 variant de 3µm à 20µm, préférablement variant de 5µm à 10 µm.

14. La composition pharmaceutique selon l'une quelconque des revendications précédentes dans laquelle la composition est une capsule, préférablement une capsule à coque dure.

15. La composition pharmaceutique selon l'une quelconque des revendications précédentes comprenant 1 mg à 4 mg de pomalidomide.

16. La composition pharmaceutique selon l'une quelconque des revendications précédentes dans laquelle le pourcentage d'ingrédient pharmaceutique n'est pas supérieur à 5,00 % en poids du contenu total de la capsule.

17. La composition pharmaceutique selon l'une quelconque des revendications précédentes destinée à être utilisée pour le traitement de cancer, préférablement de myélome multiple.
